# EUROPEAN PATENT APPLICATION

(11) **EP 1 233 063 A2**
(43) Date of publication of application: **21.08.2002**
(21) Application number: 02251132.3
(22) Date of filing: 19.02.2002
(51) Int. Cl.: C12N 15/00, A01K 67/027, C12N 9/12, C12N 5/10, A61K 49/00

(54) **Transgenic animals containing a dominant negative mutant form of the p85 subunit of pi-3 kinase**

(30) Priority: 20.02.2001 US 270014 P
(71) Applicant: Pfizer Products Inc., Groton, Connecticut 06340 (US)
(72) Inventor: Gibbs, Earl Michael, c/o Pfizer Global Res & Dev., Groton, Connecticut 06340 (US); Mcneish, John Douglas, c/o Pfizer Global Res & Dev, Groton, Connecticut 06340 (US)
(74) Representative: Hayles, James Richard

(57) **Abstract**

The present invention provides a genetically-modified, non-human mammal having an altered body fat composition, wherein said mammal comprises an exogenous mutant p85 P13-K gene, wherein the expression of said gene is driven by an insulin responsive cell specific promoter. The present invention also relates to transgenic cell lines containing the same mutant gene, as well as methods using both animals and/or cells.

Sequence Listing.ST25.txt
SEQUENCE LISTING

<110> Pfizer Products Inc.

<120> Transgenic Animals Containing a Dominant Negative Mutant Form of the
p85 Subunit of PI-3 Kinase

<130> PC10206AGPR

<160> 12

<170> PatentIn version 3.0

<210> 1
<211> 688
<212> PRT
<213> Mouse

<400> 1

Met Ser Ala Glu Gly Tyr Gln Tyr Arg Ala Leu Tyr Asp Tyr Lys Lys
1               5                   10                  15

Glu Arg Glu Glu Asp Ile Asp Leu His Leu Gly Asp Ile Leu Thr Val
            20                  25                  30

Asn Lys Gly Ser Leu Val Ala Leu Gly Phe Ser Asp Gly Pro Glu Ala
        35                  40                  45

Arg Pro Glu Asp Ile Gly Trp Leu Asn Gly Tyr Asn Glu Thr Thr Gly
    50                  55                  60

Glu Arg Gly Asp Phe Pro Gly Thr Tyr Val Glu Tyr Ile Gly Arg Lys
65                  70                  75                  80

Arg Ile Ser Pro Pro Thr Pro Lys Pro Arg Pro Pro Arg Pro Leu Pro
                85                  90                  95

Val Ala Pro Gly Ser Ser Lys Thr Glu Ala Asp Thr Glu Gln Gln Ala
            100                 105                 110

Leu Pro Leu Pro Asp Leu Ala Glu Gln Phe Ala Pro Pro Asp Val Ala
        115                 120                 125

Pro Pro Leu Leu Ile Lys Leu Leu Glu Ala Ile Glu Lys Lys Gly Leu
    130                 135                 140

Glu Cys Ser Thr Leu Tyr Arg Thr Gln Ser Ser Ser Asn Pro Ala Glu
145                 150                 155                 160

Leu Arg Gln Leu Leu Asp Cys Asp Ala Ala Ser Val Asp Leu Glu Met
                165                 170                 175

Sequence Listing.ST25.txt
Ile Asp Val His Val Leu Ala Asp Ala Phe Lys Arg Tyr Leu Ala Asp
            180                 185                 190

Leu Pro Asn Pro Val Ile Pro Val Ala Val Tyr Asn Glu Met Met Ser
        195                 200                 205

Leu Ala Gln Glu Leu Gln Ser Pro Glu Asp Cys Ile Gln Leu Leu Lys
    210                 215                 220

Lys Leu Ile Arg Leu Pro Asn Ile Pro His Gln Cys Trp Leu Thr Leu
225                 230                 235                 240

Gln Tyr Leu Leu Lys His Phe Phe Lys Leu Ser Gln Ala Ser Ser Lys
                245                 250                 255

Asn Leu Leu Asn Ala Arg Val Leu Ser Glu Ile Phe Ser Pro Val Leu
            260                 265                 270

Phe Arg Phe Pro Ala Ala Ser Ser Asp Asn Thr Glu His Leu Ile Lys
        275                 280                 285

Ala Ile Glu Ile Leu Ile Ser Thr Glu Trp Asn Glu Arg Gln Pro Ala
    290                 295                 300

Pro Ala Leu Pro Pro Lys Pro Pro Lys Pro Thr Thr Val Ala Asn Asn
305                 310                 315                 320

Ser Met Asn Asn Asn Met Ser Leu Gln Asp Ala Glu Trp Tyr Trp Gly
                325                 330                 335

Asp Ile Ser Arg Glu Glu Val Asn Glu Lys Leu Arg Asp Thr Ala Asp
            340                 345                 350

Gly Thr Phe Leu Val Arg Asp Ala Ser Thr Lys Met His Gly Asp Tyr
        355                 360                 365

Thr Leu Thr Leu Arg Lys Gly Gly Asn Asn Lys Leu Ile Lys Ile Phe
    370                 375                 380

His Arg Asp Gly Lys Tyr Gly Phe Ser Asp Pro Leu Thr Phe Asn Ser
385                 390                 395                 400

Val Val Glu Leu Ile Asn His Tyr Arg Asn Glu Ser Leu Ala Gln Tyr
                405                 410                 415

Asn Pro Lys Leu Asp Val Lys Leu Leu Tyr Pro Val Ser Lys Tyr Gln
            420                 425                 430

Gln Asp Gln Val Val Lys Glu Asp Asn Ile Glu Ala Val Gly Lys Lys
        435                 440                 445

Leu His Glu Tyr Asn Thr Gln Phe Gln Glu Lys Ser Arg Glu Tyr Asp
    450                 455                 460

Sequence Listing.ST25.txt

Arg Leu Tyr Glu Glu Tyr Thr Arg Thr Ser Gln Glu Ile Arg Glu Gly
465                 470                 475                 480

Asn Glu Lys Glu Ile Gln Arg Ile Met His Asn His Asp Lys Leu Lys
                485                 490                 495

Ser Arg Ile Ser Glu Ile Ile Asp Ser Arg Arg Arg Leu Glu Glu Asp
            500                 505                 510

Leu Lys Lys Gln Ala Ala Glu Tyr Arg Glu Ile Asp Lys Arg Met Asn
        515                 520                 525

Ser Ile Lys Pro Asp Leu Ile Gln Leu Arg Lys Thr Arg Asp Gln Tyr
    530                 535                 540


545                 550                 555                 560


                565                 570                 575


            580                 585                 590


        595                 600                 605


    610                 615                 620


625                 630                 635                 640


                645                 650                 655


            660                 665                 670


        675                 680                 685

<210>  2
<211>  6591
<212>  DNA
<213>  Artificial Sequence

<400>  2
tggagctcca ccgcggtggc ggccgctcta gatgcatgct cgagcggccg ccagtgtgat      60

ggatatctgc agaattcggc ttgagtctag atcactctgt cgccaggctg gagtgcaagg     120

Sequence Listing.ST25.txt
gcacgatctt ggctcactac aacctccacc tcctgggttc aagccatttt cctgcctcag 180

cctcccgagt agctgggatt acaggtgtgc ataaccacgc ccggctaatt tttgtatctt 240

tagcagacat ggggtttctc tatgttggcc aggctggttt caaactcctg acctcagtcg 300

atccacctgc cttggcctcc taaagtgctg ggattacagg catgagccac caggccgggc 360

cggcattcca gatttttcag gggattcgtg cagcaaagga atcaagaagg gatgtaaagg 420

cacagtgtgt tctgggtaca ataaggactt aggcattgcc cagaacagga ggcgaaggag 480

atagaaggag aggcaggaga gataggtaag gccagagatc ggataagaga ggcaggaggt 540

tttgttcact ctgaaaaggg atttgaactt ggcaattggg gcaacagaga cagtgacttc 600

ttgcttgaga gatgagattg gaccttcgaa aattgttctc tgccctcgtc ataaaggaaa 660

taagaggagc acgaagacca gtgagggtga tggtgatctg gactgaagtg gcagccgcca 720

cggagaatat cggatgaatg tgagagagtt ttggaggtca aagcaccaat gttggaaact 780

aactggataa acgaggagag cggcgcagga caggaggaat cgagcctgac ttctaccata 840

ggggtgactg ggcgggtaat tcattgaaat aaggaagtta ggaggaggag caggtttgga 900

catgctgatc actagagctg ccacatccgg gcggtaacga acacctggat ctgcagctcc 960

agagaagggc ctgggtcaga tgtcactgaa gccctatggt ggcggaaagg cgagaaatag 1020

tgggttgaga ttccaagtgc aatccactgc ggctcctcgc tcgccctcca ggtggcagca 1080

caaccctgcg cttccgaagc ccgttttctg agccagacac tctccacgct ctgggtattt 1140

cggcttctct ctccccacac gccgacccta ggtcgcgcac tttctgcctg gcagaatttg 1200

gccgaggatc caaacccgga gcagcctcca gagagcgtgt cgttcacgcg gccagcatat 1260

gctcagagac ctcagaggct cagagacctc agggctggtg gtgtggtcgg ttgtgaccac 1320

ttgtccctcg gaccggctcc aggaaccaac ctggggaatg tgtgtagggg aagggcggga 1380

tagacagtgc ccggagcagg gaggcgctga aagacaggac caagcagccc ggccaccaga 1440

cccgttgtgg gaacggaatt tcctggcccc cagggccaca ctcgcgtggg aagcatgtcg 1500

cggacccttt aaggcgtcat ctccctgtct ctccgccccc gcctgggaca ggccgggacg 1560

cccgggacct gacatttgga ggctcccaac gtgggagcta aaaatagcag ccccgggtta 1620

ctttggggca ttgctcctct cccaacccgc gcgccggctc gcgagccgtc tcaggccgct 1680

ggagtttccc cggggcaagt acacctggcc cgtcctctcc tctcagaccc cactgtccag 1740

Sequence Listing.ST25.txt

acccgcagag tttaagatgc ttctgcagcc cgggatccta gctggtgggc ggagtcctaa 1800

cacgtgggtg ggcggggcct tttgttccag ggactctttt ctcaaaactt cccagtcgga 1860

ggctggcggg aacccgagag gcgtgtctcg ccagccacgc ggaggggcgt ggcctcattg 1920

gcccgcccca ccaactccag ccaaactcta aaccccaggc ggagggggcg tggccttctg 1980

gggtgtgcgg gctcctggcc aatgggtgct gtgaagggcg tggcccgcgg gggcaggagc 2040

gaggtggcgg gggcttctcg cgtcttttcc cccagccccg ctccacaaga tccgcgggag 2100

ccccactgct ctccggatcc ttggcttgtg gctgtgggtc ccatcggact ctagagtggc 2160

agggcactag agctgcagac atgagtgcag agggctacca gtacagagca ctgtacgact 2220

acaagaagga gcgagaggaa gacattgacc tacacctggg ggacatactg actgtgaata 2280

aaggctcctt agtggcactt ggattcagtg atggccagga agcccggcct gaagatattg 2340

gctggttaaa tggctacaat gaaaccactg gggagagggg agactttcca ggaacttacg 2400

ttgaatacat tggaaggaaa agaatttcac cccctactcc caagcctcgg ccccctcgac 2460

cgcttcctgt tgctccgggt tcttcaaaaa ctgaagctga cacggagcag caagcgttgc 2520

cccttcctga cctggccgag cagtttgccc ctcctgatgt tgccccgcct ctccttataa 2580

agctcctgga agccattgag aagaaaggac tggaatgttc gactctatac agaacacaaa 2640

gctccagcaa ccctgcagaa ttacgacagc ttcttgattg tgatgccgcg tcagtggact 2700

tggagatgat cgacgtacac gtcttagcag atgctttcaa acgctatctc gccgacttac 2760

caaatcctgt cattctgtag ctgtttacaa tgagatgatg tctttagccc aagaactaca 2820

gagccctgaa gactgcatcc agctgttgaa gaagctcatt agattgccta atatacctca 2880

tcagtgttgg cttacgcttc agtatttgct caagcatttt ttcaagctct ctcaagcctc 2940

cagcaaaaac cttttgaatg caagagtcct ctctgagatt ttcagccccg tgcttttcag 3000

atttccagcc gccagctctg ataatactga acacctcata aaagcgatag agattttaat 3060

ctcaacggaa tggaatgaga gacagccagc accagcactg ccccccaaac cacccaagcc 3120

cactactgta gccaacaaca gcatgaacaa caatatgtcc ttgcaggatg ctgaatggta 3180

ctggggagac atctcaaggg aagaagtgaa tgaaaaactc cgagacactg ctgatgggac 3240

ctttttggta cgagacgcat ctactaaaat gcacggcgat tacactctta cactaaggaa 3300

Sequence Listing.ST25.txt
aggaggaaat aacaaattaa tcaaaatctt tcaccgtgat ggaaaatatg gcttctctga 3360
tccattaacc ttcaactctg tggttgagtt aataaaccac taccggaatg agtctttagc 3420
tcagtacaac cccaagctgg atgtgaagtt gctctaccca gtgtccaaat accagcagga 3480
tcaagttgtc aaagaagata atattgaagc tgtagggaaa aaattacatg aatataatac 3540
tcaatttcaa gaaaaaagtc gggaatatga tagattatat gaggagtaca cccgtacttc 3600
ccaggaaatc agatctcgcg aaggcaacga gaaagaaatt caaaggatta tgcataacca 3660
tgataagctg aagtcgcgta tcagtgagat cattgacagt aggaggaggt tggaagaaga 3720
cttgaagaag caggcagctg agtaccgaga gatcgacaaa cgcatgaaca gtattaagcc 3780
ggacctcatc cagttgagaa agacaagaga ccaatacttg atgtggctga cgcagaaagg 3840
tgtgcggcag aagaagctga acgagtggct ggggaatgaa aataccgaag atcaatactc 3900
cctggtagaa gatgatgagg atttgcccca ccatgacgag aagacgtgga atgtcgggag 3960
cagcaaccga aacaaagcgg agaacctatt gcgagggaag cgagacggca ctttccttgt 4020
ccgggagagc agtaagcagg gctgctatgc ctgctccgta gtggtagacg gcgaagtcaa 4080
gcattgcgtc attaacaaga ctgccaccgg ctatggcttt gccgagccct acaacctgta 4140
cagctccctg aaggagctgg tgctacatta tcaacacacc tccctcgtgc agcacaatga 4200
ctccctcaat gtcacactag catacccagt atatgcacaa cagaggcgga tccccccccg 4260
gggaggcctg tcgcgatccg gaccggaaca aaagcttatt tctgaagaag acttggctag 4320
cagtactcgc cggcccccgg gggctgcagg aattccgatg atcccaaggc ccaactcccc 4380
gaaccactca gggtcctgtg gacagctcac ctagcggcaa tggctacagg taagcgcccc 4440
taaaatccct ttgggcacaa tgtgtcctga ggggagaggc agcgacctgt agatgggacg 4500
ggggcactaa ccctcaggtt tggggcttct gaatgtgagt atcgccatgt aagcccagta 4560
tttggccaat ctcagaaagc tcctggtccc tggagggatg gagagagaaa aacaaacagc 4620
tcctggagca gggagagtgc tggcctcttg ctctccggct ccctctgttg ccctctggtt 4680
tctccccagg ctcccggacg tccctgctcc tggcttttgg cctgctctgc ctgccctggc 4740
ttcaagaggg cagtgccttc ccaaccattc ccttatccag gctttttgac aacgctatgc 4800
tccgcgccca tcgtctgcac cagctggcct ttgacaccta ccaggagttt gtaagctctt 4860
ggggaatggg tgcgcatcag gggtggcagg aaggggtgac tttcccccgc tgggaaataa 4920

Sequence Listing.ST25.txt

gaggaggaga ctaaggagct cagggttttt cccgaagcga aaatgcaggc agatgagcac 4980

acgctgagtg aggttcccag aaaagtaaca atgggagctg gtctccagcg tagaccttgg 5040

tgggcggtcc ttctcctagg aagaagccta tatcccaaag gaacagaagt attcattcct 5100

gcagaacccc cagacctccc tctgtttctc agagtctatt ccgacaccct ccaacaggga 5160

ggaaacacaa cagaaatccg tgagtggatg ccttctcccc aggcggggat gggggagacc 5220

tgtagtcaga gcccccgggc agcacagcca atgcccgtcc ttcccctgca gaacctagag 5280

ctgctccgca tctccctgct gctcatccag tcgtggctgg agcccgtgca gttcctcagg 5340

agtgtcttcg ccaacagcct ggtgtacggc gcctctgaca gcaacgtcta tgacctccta 5400

aaggacctag aggaaggcat ccaaacgctg atgggggtga gggtggcgcc aggggtcccc 5460

aatcctggag ccccactgac tttgagagct gtgttagaga aacactgctg ccctcttttt 5520

agcagtcagg ccctgaccca agagaactca ccttattctt catttcccct cgtgaatcct 5580

ccaggccttt ctctacaccc tgaaggggag ggaggaaaat gaatgaatga gaaagggagg 5640

gaacagtacc caagcgcttg gcctctcctt ctcttccttt cactttgcag aggctggaag 5700

atggcagccc ccggactggg cagatcttca agcagaccta cagcaagttc gacacaaact 5760

cacacaacga tgacgcacta ctcaagaact acgggctgct ctactgcttc aggaaggaca 5820

tggacaaggt cgagacattc ctgcgcatcg tgcagtgccg ctctgtggag ggcagctgtg 5880

gcttctagct gcccgggtgg catccctgtg acccctcccc agtgcctctc ctggccctgg 5940

aagttgccac tccagtgccc accagccttg tcctaataaa attaagttgc atcattttgt 6000

ctgactaggt gtccttctat aatattatgg ggtggagggg ggtggtatgg agcaaggggc 6060

aagttgggaa gacaacctgt agggcctgcg gggtctattg ggaaccaagc tggagtgcag 6120

tggcacaatc ttggctcact gcaatctccg cctcctgggt tcaagcgatt ctcctgcctc 6180

agcctcccga gttgttggga ttccaggcat gcatgaccag gctcagctaa tttttgtttt 6240

tttggtagag acggggtttc accatattgg ccaggctggt ctccaactcc taatctcagg 6300

tgatctaccc accttggcct cccaaattgc tgggattaca ggcgtgaacc actgctccct 6360

tccctgtcct tctgatttta aaataactat accagcagga ggacgtccag acacagcata 6420

ggctacctgg ccatgcccaa ccggtgggac atttgagttg cttgcttggc actgtcctct 6480

Sequence Listing.ST25.txt
catgcgttgg gtccactcag tagatgcctg ttgaattcga tatcaagctt atcgataccg 6540

tcgacctcga gggggggccc ggtacccaat tcgccctata gtgagtcgta t 6591

<210> 3
<211> 724
<212> PRT
<213> Mouse

<400> 3

Met Ser Ala Glu Gly Tyr Gln Tyr Arg Ala Leu Tyr Asp Tyr Lys Lys
1               5                   10                  15

Glu Arg Glu Glu Asp Ile Asp Leu His Leu Gly Asp Ile Leu Thr Val
            20                  25                  30

Asn Lys Gly Ser Leu Val Ala Leu Gly Phe Ser Asp Gly Pro Glu Ala
        35                  40                  45

Arg Pro Glu Asp Ile Gly Trp Leu Asn Gly Tyr Asn Glu Thr Thr Gly
    50                  55                  60

Glu Arg Gly Asp Phe Pro Gly Thr Tyr Val Glu Tyr Ile Gly Arg Lys
65                  70                  75                  80

Arg Ile Ser Pro Pro Thr Pro Lys Pro Arg Pro Pro Arg Pro Leu Pro
                85                  90                  95

Val Ala Pro Gly Ser Ser Lys Thr Glu Ala Asp Thr Glu Gln Gln Ala
            100                 105                 110

Leu Pro Leu Pro Asp Leu Ala Glu Gln Phe Ala Pro Pro Asp Val Ala
        115                 120                 125

Pro Pro Leu Leu Ile Lys Leu Leu Glu Ala Ile Glu Lys Lys Gly Leu
    130                 135                 140

Glu Cys Ser Thr Leu Tyr Arg Thr Gln Ser Ser Ser Asn Pro Ala Glu
145                 150                 155                 160

Leu Arg Gln Leu Leu Asp Cys Asp Ala Ala Ser Val Asp Leu Glu Met
                165                 170                 175

Ile Asp Val His Val Leu Ala Asp Ala Phe Lys Arg Tyr Leu Ala Asp
            180                 185                 190

Leu Pro Asn Pro Val Ile Pro Val Ala Val Tyr Asn Glu Met Met Ser
        195                 200                 205

Leu Ala Gln Glu Leu Gln Ser Pro Glu Asp Cys Ile Gln Leu Leu Lys
    210                 215                 220

Sequence Listing.ST25.txt

Lys Leu Ile Arg Leu Pro Asn Ile Pro His Gln Cys Trp Leu Thr Leu
225                 230                 235                 240

Gln Tyr Leu Leu Lys His Phe Phe Lys Leu Ser Gln Ala Ser Ser Lys
                245                 250                 255

Asn Leu Leu Asn Ala Arg Val Leu Ser Glu Ile Phe Ser Pro Val Leu
            260                 265                 270

Phe Arg Phe Pro Ala Ala Ser Ser Asp Asn Thr Glu His Leu Ile Lys
        275                 280                 285

Ala Ile Glu Ile Leu Ile Ser Thr Glu Trp Asn Glu Arg Gln Pro Ala
    290                 295                 300

Pro Ala Leu Pro Pro Lys Pro Pro Lys Pro Thr Thr Val Ala Asn Asn
305                 310                 315                 320

Ser Met Asn Asn Asn Met Ser Leu Gln Asp Ala Glu Trp Tyr Trp Gly
                325                 330                 335

Asp Ile Ser Arg Glu Glu Val Asn Glu Lys Leu Arg Asp Thr Ala Asp
            340                 345                 350

Gly Thr Phe Leu Val Arg Asp Ala Ser Thr Lys Met His Gly Asp Tyr
        355                 360                 365

Thr Leu Thr Leu Arg Lys Gly Gly Asn Asn Lys Leu Ile Lys Ile Phe
    370                 375                 380

His Arg Asp Gly Lys Tyr Gly Phe Ser Asp Pro Leu Thr Phe Asn Ser
385                 390                 395                 400

Val Val Glu Leu Ile Asn His Tyr Arg Asn Glu Ser Leu Ala Gln Tyr
                405                 410                 415

Asn Pro Lys Leu Asp Val Lys Leu Leu Tyr Pro Val Ser Lys Tyr Gln
            420                 425                 430

Gln Asp Gln Val Val Lys Glu Asp Asn Ile Glu Ala Val Gly Lys Lys
        435                 440                 445

Leu His Glu Tyr Asn Thr Gln Phe Gln Glu Lys Ser Arg Glu Tyr Asp
    450                 455                 460

Arg Leu Tyr Glu Glu Tyr Thr Arg Thr Ser Gln Glu Ile Gln Met Lys
465                 470                 475                 480

Arg Thr Ala Ile Glu Ala Phe Asn Glu Thr Ile Lys Ile Phe Glu Glu
                485                 490                 495

Gln Cys Gln Thr Gln Glu Arg Tyr Ser Lys Glu Tyr Ile Gly Lys Phe

Sequence Listing.ST25.txt
            500                 505                 510

Lys Arg Glu Gly Asn Glu Lys Glu Ile Gln Arg Ile Met His Asn His
        515                 520                 525

Asp Lys Leu Lys Ser Arg Ile Ser Glu Ile Ile Asp Ser Arg Arg Arg
    530                 535                 540

Leu Glu Glu Asp Leu Lys Lys Gln Ala Ala Glu Tyr Arg Glu Ile Asp
545                 550                 555                 560

Lys Arg Met Asn Ser Ile Lys Pro Asp Leu Ile Gln Leu Arg Lys Thr
                565                 570                 575

Arg Asp Gln Tyr Leu Met Trp Leu Thr Gln Lys Gly Val Arg Gln Lys
            580                 585                 590

Lys Leu Asn Glu Trp Leu Gly Asn Glu Asn Thr Glu Asp Gln Tyr Ser
        595                 600                 605

Leu Val Glu Asp Asp Glu Asp Leu Pro His His Asp Glu Lys Thr Trp
    610                 615                 620

Asn Val Gly Ser Ser Asn Arg Asn Lys Ala Glu Asn Leu Leu Arg Gly
625                 630                 635                 640

Lys Arg Asp Gly Thr Phe Leu Val Arg Glu Ser Ser Lys Gln Gly Cys
                645                 650                 655

Tyr Ala Cys Ser Val Val Val Asp Gly Glu Val Lys His Cys Val Ile
            660                 665                 670

Asn Lys Thr Ala Thr Gly Tyr Gly Phe Ala Glu Pro Tyr Asn Leu Tyr
        675                 680                 685

Ser Ser Leu Lys Glu Leu Val Leu His Tyr Gln His Thr Ser Leu Val
    690                 695                 700

Gln His Asn Asp Ser Leu Asn Val Thr Leu Ala Tyr Pro Val Tyr Ala
705                 710                 715                 720

Gln Gln Arg Arg


<210>  4
<211>  22
<212>  DNA
<213>  Artificial Sequence

<400>  4
agctctagag tggcagggca ct                                                22

Sequence Listing.ST25.txt
<210> 5
<211> 23
<212> DNA
<213> Artifical Sequence

<400> 5
tttagatctg atttcctggg aag 23


<210> 6
<211> 22
<212> DNA
<213> Artificial Sequence

<400> 6
aagagatctc gcgaaggcaa cg 22


<210> 7
<211> 25
<212> DNA
<213> Artificial Sequence

<400> 7
cagcgcggat ccgcctctgt tgtgc 25


<210> 8
<211> 85
<212> DNA
<213> Artificial Sequence

<400> 8
cccggggagg cctgtcgcga tccggaccgg aacaaaagct tatttctgaa gaagacttgg 60

ctagcagtac tcgccggccc ccggg 85


<210> 9
<211> 20
<212> DNA
<213> Artificial Sequence

<400> 9
gagtctagat cactctgtcg 20


<210> 10
<211> 18
<212> DNA
<213> Artificial Sequence

<400> 10

Sequence Listing.ST25.txt
gactctagag tccgatgg                                                    18


<210>  11
<211>  2064
<212>  DNA
<213>  Artificial Sequence

<400>  11
gagacggagt cactctgtcg ccaggctgga gtgcaagggc acgatcttgg ctcactacaa      60

cctccacctc ctgggttcaa gccattttcc tgcctcagcc tcccgagtag ctgggattac     120

aggtgtgcat aaccacgccc ggctaatttt tgtatcttta gcagacatgg ggtttctcta     180

tgttggccag gctggtttca aactcctgac ctcagtcgat ccacctgcct tggcctccta     240

aagtgctggg attacaggca tgagccacca ggccgggccg gcattccaga tttttcaggg     300

gattagtgca gcaaaggaat caagaaggga tgtaaaggca cagtgtgttc tgggtacaat     360

aaggacttag gcattgccca gagcaggagg cgaaggagat agaaggagag gcaggagaga     420

taggtaaggc cagagatcgg ataagagagg caggaggttt tgttcactct gaaaagggat     480

ttgaacttgg caattggggc aacagagaca gtgacttctt gcttgagaga tgagattgga     540

ccttcgaaaa ttgttctctg ccctcgtcat aaaggaaata agaggagcac gaagaccagt     600

gagggtgatg gtgatctgga ctgaagtggc agccgccacg gagaatatcg gatgaatgtg     660

agagagtttt ggaggtcaaa gcaccaatgt tggaaactaa ctggataaac gaggagagcg     720

gcgcaggaca ggaggaatcg agcctgactt ctaccatagg ggtgactggg cgggtaattc     780

attgaaataa ggaagttagg aggaggagca ggtttggaca tgctgatcac tagagctgcc     840

acatccgggc ggtaacgaac acctggatct gcagctccag agaagggcct gggtcagatg     900

tcactgaagc cctatggtgg cggaaaggcg agaaatagtg ggttgagatt ccaagtgcaa     960

tccactgcgg ctcctcgctc gccctccagg tggcagcaca accctgcgct tccgaagccc    1020

gttttctgag ccagacactc tccacgctct gggtatttcg gcttctctct ccccacacgc    1080

cgaccctagg tcgcgcactt tctgcctggc agaatttggc cgaggatcca aacccggagc    1140

agcctccaga gagcgtgtcg ttcacgcggc cagcatatgc tcagagacct cagaggctca    1200

gagacctcag ggctggtggt gtggtcggtt gtgaccactt gtccctcgga ccggctccag    1260

gaaccaacct ggggaatgtg tgtaggggaa gggcgggata gacagtgccc ggagcaggga    1320

Sequence Listing.ST25.txt
ggcgctgaaa gacaggacca agcagcccgg ccaccagacc cgttgtggga acggaatttc 1380

ctggccccca gggccacact cgcgtgggaa gcatgtcgcg gaccctttaa ggcgtcatct 1440

ccctgtctct ccgcccccgc ctgggacagg cgggacgccc gggacctgac atttggaggc 1500

tcccaacgtg ggagctaaaa atagcagccc cgggttactt tggggcattg ctcctctccc 1560

aacccgcgcg ccggctcgcg agccgtctca ggccgctgga gtttccccgg ggcaagtaca 1620

cctggcccgt cctctcctct cagaccccac tgtccagacc cgcagagttt aagatgcttc 1680

tgcagcccgg gatcctagct ggtgggcgga gtcctaacac gtgggtgggc ggggcctttt 1740

gttccaggga ctcttttctc aaaacttccc agtcggaggc tggcgggaac ccgagaggcg 1800

tgtctcgcca gccacgcgga ggggcgtggc ctcattggcc cgccccacca actccagcca 1860

aactctaaac cccaggcgga gggggcgtgg ccttctgggg tgtgcgggct cctggccaat 1920

gggtgctgtg aagggcgtgg cccgcggggg caggagcgag gtggcggggg cttctcgcgt 1980

cttttccccc agccccgctc caccagatcc gcgggagccc cactgctctc cgggtccttg 2040

gcttgtggct gtgggtccca tcgg 2064

<210> 12
<211> 18
<212> DNA
<213> Artificial Sequence

<400> 12
ccggaattct gatagtaa 18

## Description

### FIELD OF THE INVENTION

The present invention relates to transgenic animals containing a dominant negative mutant form of the regulatory p85 subunit of phosphatidylinositol 3-kinase. Surprisingly, rather than being a diabetic phenotype, these animals exhibit an altered body fat composition, having a marked increase in body fat coupled with a corresponding decrease in lean muscle mass. The present invention also relates to transgenic cell lines containing the same mutant gene, as well as methods for using both animals and/or cells.

### BACKGROUND OF THE INVENTION

Many growth factors and hormones such as nerve growth factor, platelet derived growth factor, epidermal growth factor, and insulin mediate their signals through interactions with cell surface tyrosine kinase receptors. The transduction of extracellular signals across the membrane, initiated by ligand binding, leads to the propagation of multiple signaling events, which ultimately control target biochemical pathways within the cell.

The phosphatidylinositol 3-kinases (PISKs) represent a ubiquitous family of heterodimeric lipid kinases that are found in association with the cytoplasmic domain of hormone and growth factor receptors and oncogene products. PI3Ks act as downstream effectors of these receptors, are recruited upon receptor stimulation and mediate the activation of second messenger signaling pathways through the production of phosphorylated derivatives of inositol (Fry, *Biochim. Biophys. Acta.,* 1226:237-68 (1994)).

PI3Ks have been implicated in many cellular activities including growth factor mediated cell transformation, mitogenesis, protein trafficking, cell survival and proliferation, DNA synthesis, apoptosis, neurite outgrowth, and insulin-stimulated glucose transport (reviewed in Fry, Id.).

The PI3 kinase enzyme heterodimers consist of a 110 kD (p110) catalytic subunit associated with an 85 kD (p85) regulatory subunit and it is through the SH2 domains of the p85 subunit that the enzyme associates with the membrane-bound receptors (Escobedo et al., *Cell,* 65:75-82(1991); Skolnik et al., *Cell,* 65:83-90 (1991)).

PI3K p85 (also known as GRBI and PIK3R1) was initially isolated from bovine brain and shown to exist in two forms, α and β. in these studies p85 isoforms were shown to bind to and act as substrates for tyrosine-phosphorylated receptor kinases and the polyoma virus middle T antigen complex (Otsu et al., *Cell,* 65:91-104 (1991)). Since then the p85 subunit has been further characterized and shown to interact with a diverse group of proteins including tyrosine kinase receptors such as the erythropoietin receptor, the PDGR-β receptor, and Tie2, an endothelium-specific receptor involved in vascular development and tumor angiogenesis (Escobedo et al., *Cell,* 65:75-82 (1991); He et al., *Blood,* 82:3530-38 (1993); Kontos et al., *Mol. Cell. Biol.,* 18:4131-40 (1998)). It also interacts with focal adhesion kinase (FAK), a cytoplasmic tyrosine kinase involved in integrin signaling and is thought to be a substrate and effector of FAK. The p85 subunit also interacts with profilin, an actin binding protein that facilitates actin polymerization (Bhargavi et al., *Biochem. Mol. Biol. Int*., 46:241-248 (1998); Chen and Guan, *PNAS,* 91:10148-52 (1994)) and the p85/profilin complex inhibits actin polymerization.

Recently, a truncated form of the PI3K p85 subunit was isolated (Jimenez et al., *Embo J.,* 17:743-53 (1998)). This form includes the first 571 amino acids of the wild type (encoded by nucleotides 43-1755 of Genbank Act. No. M61906) linked to a region that is conserved in the eph tyrosine kinase receptor family. This truncation was shown to induce the constitutive activation of PI3 kinase and contribute to cellular transformation of mammalian fibroblasts.

Several chemically distinct inhibitors for PI3 kinases are reported in the literature. These include wortmannin, a fungal metabolite (Ui et al., *Trends Biochem. Sci.,* 20:303-07 (1995)), demethoxyviridin, an antifungal agent (Woscholski et al., *FEBS Lett*., 342:109-14 (1994)), and quercetin and LY294002, two related chromones (Vlahos et al., *J. Biol. Chem.,* 269:5241-48 (1994)). However, these inhibitors primarily target the p110 subunit.

### SUMMARY OF THE INVENTION

Insulin activates PI3-K by stimulating the tyrosine phosphorylation of IRS-1 and IRS-2 which then bind to p85 via SH2 domains and results in the rapid activation of the p110 catalytic domain. Thus, overexpression of mutant p85 was expected to lead to a situation in which PI3-K is not stimulatable by insulin because most of the phospho-IRS-1 and phospho-IRS-2, which normally binds to p85 and activates PI3-K, will be complexed with mutant p85 molecules.

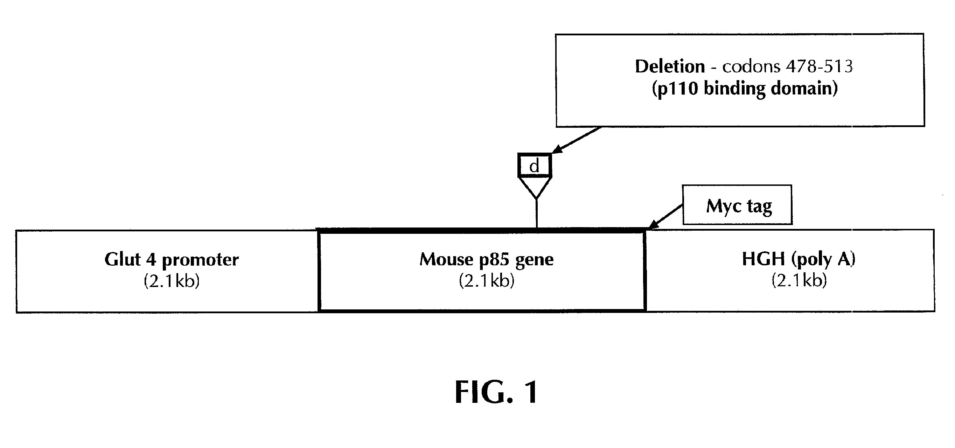

Thus, to achieve the present invention, a DNA expression vector was produced containing a truncated mouse p85 gene encoding a protein that contains the two SH2 domains, but lacks the inter-SH2 domain required for binding p110. This mutant gene was placed under the control of the muscle and fat cell specific Glut4 promoter/enhancer (see Figure 1). This promoter consists of 2.1kb of 5' Glut4 flanking DNA that contains the cis-acting DNA sequences required for tissue-specific expression of the Glut4 protein (Olson et al., *J. Biol. Chem.,* 268:9839-46 (1993)). The Glut4 promoter/enhancer element has been shown to direct high level gene expression of the Glut4 protein (Id.; and Gibbs et al., *J. Clin. Invest.,* 95:1512-18 (1995)) as well as a chloramphenicol acetyltransferase (CAT) reporter gene (Olson, Id.). Successful expression with the CAT construct indicates that elements within the Glut4 coding sequence are not required for promoter activity. Thus, transgenic animals and cells may be produced containing this DNA construct.

In a first aspect, the present invention provides a genetically-modified, non-human mammal having an altered body fat composition, wherein said mammal comprises an exogenous mutant p85 PI3-K gene, wherein the expression of said gene is driven by an insulin responsive cell specific promoter. In a preferred embodiment, the mammal is a mouse comprising an exogenous mutant p85 PI3-K gene having the amino acid sequence of SEQ ID NO:1, wherein the expression of said gene is driven by the Glut4 promoter.

In a second aspect, the present invention provides a genetically-modified animal cell, wherein said cell comprises an exogenous mutant p85 PI3-K gene, wherein the expression of said gene is driven by an insulin responsive cell specific promoter. In a preferred embodiment, the cell is a mouse cell comprising an exogenous mutant p85 PI3-K gene having the amino acid sequence of SEQ ID NO:1, wherein the expression of said gene is driven by the Glut4 promoter.

In a third aspect, the present invention provides a method of identifying an agent that modulates the body fat composition of a mammal, said method comprising the steps of comparing the body fat compositions of a test animal to a control animal, wherein said agent has been administered to said test animal, further wherein each of said test and control animals is a genetically-modified, non-human mammal having an increased body fat composition, wherein said mammal comprises an exogenous mutant p85 PI3-K gene, wherein the expression of said gene is driven by an insulin responsive cell specific promoter, and identifying as a modulating agent the agent that has a statisticaiiy significant effect on the body fat ' composition of the test animal.

In a fourth aspect, the present invention provides a DNA expression construct for expressing a mutant p85 PI3-K gene, said construct comprising a mutant p85 PI3-K gene, wherein said gene encodes a p85 PI3-K protein whose inter-SH2 domain is unable to functionally interact with the p110 subunit of PI3-K, and an insulin responsive cell specific promoter that controls the expression of said gene. In a preferred embodiment, the construct comprises a mutant p85 PI3-K gene, wherein said gene encodes a mutant p85 PI3-K protein having the amino acid sequence of SEQ ID NO:1, and a Glut4 promoter that controls the expression of said gene.

### DESCRIPTION OF THE DRAWINGS

Figure 1 provides a representation of the mutant p85 dominant negative transgenic DNA construct used to create the mutant cells and mammal lines of the present invention. The nucleotide sequence of a preferred embodiment of this DNA construct is seen in SEQ ID NO:2.
Figure 2 provides a table showing a comparison of the fat, lean muscle, and total weight compositions of wild-type mice and mice produced according to the present invention.
Figure 3 provides a graphical presentation of the body fat percentages over time of wild-type mice and mice produced according to the present invention.

### DETAILED DESCRIPTION

Those skilled in the art will fully understand the terms used herein in the description and the appendant claims to describe the present invention. Nonetheless, unless otherwise provided herein, the following terms are as described immediately below.

A non-human mammal or an animal cell that is "genetically-modified" is heterozygous or homozygous for a modification that is introduced into the non-human mammal or animal cell, or into a progenitor non-human mammal or animal cell, by genetic engineering. The standard methods of genetic engineering that are available for introducing the modification include homologous recombination, viral vector gene trapping, irradiation, chemical mutagenesis, and the transgenic expression of a nucleotide sequence encoding antisense RNA alone or in combination with catalytic ribozymes. Preferred methods for genetic modification are those which modify an endogenous gene by inserting a "foreign nucleic acid sequence" into the gene locus, e.g., homologous recombination and viral vector gene trapping. The most preferred method of genetic engineering is homologous recombination, in which a foreign nucleic acid sequence is inserted in a targeted manner either alone or in combination with specific nucleotide changes to, or a deletion of, a portion of the endogenous gene sequence.

By "PI3-K" is meant the protein phosphatidylinositol 3-kinase, or sometimes the gene encoding the PI3-K protein. Identical terms are "PI3K" and "P13-kinase", and "PI3 kinase". PI3-K is composed of the p85 regulatory subunit and the p110 enzymatic subunit. Preferably, the mouse p85 subunit of PI3-K has the amino acid sequence provided in Escobedo et al., *Cell,* 65:75-82 (1991); see also GenBank Acc. No. M60651 and SEQ ID NO:3.

By a "mutant p85" or "mp85" is meant a gene encoding the p85 subunit that is genetically-modified such that the polypeptide encoded by the mutant gene is unable to bind to and interact properly with the p110 subunit. Thus, because the p85 subunit is mutated, it does not engage fully in its normal activity of binding to and regulating the activity of the p110 subunit. Preferably, the mp85 polypeptide lacks the inter-SH2 domain, although other genetic modifications that disable the ability of mp85 to regulate p110 are contemplated.

By "inter-SH2 domain" is meant that portion of the p85 subunit that is necessary for binding of the p85 subunit to the p110 subunit, and which enables the p85 subunit to properly regulate the p110 subunit. In GenBank Acc. No. M60651, the inter-SH2 domain corresponds approximately to nucleotides 1988-2095; in SEQ ID NO:3, the inter-SH2 domain corresponds approximately to amino acids 478-513.

By a "genetically-modified, non-human mammal" containing a mutant p85 gene is meant a non-human mammal that is produced, for example, by creating a blastocyst carrying the desired genetic modification and then implanting the blastocyst in a foster mother for *in utero* development. The genetically-modified blastocyst can be made, in the case of mice, by implanting a genetically-modified embryonic stem (ES) cell into a mouse blastocyst. Alternatively, various species of genetically-modified embryos can be obtained by nuclear transfer. In the case of nuclear transfer, the donor cell is a somatic ceii or a piuripotent stem cell, and it is engineered to contain the desired genetic modification. The nucleus of this cell is then transferred into a fertilized or parthenogenetic oocyte that is enucleated, the embryo is reconstituted, and developed into a blastocyst. A genetically-modified blastocyst produced by either of the above methods is then implanted into a foster mother according to standard methods known to those of skill in the art. A "genetically-modified, non-human mammal" includes all progeny of the mammals created by the methods described above, provided that the progeny inherit at least one copy of the introduced genetic modification. It is preferred that all somatic cells and germline cells of the genetically-modified mammal contain the modification. Preferred non-human animals that are genetically-modified include rodents, such as mice and rats, rabbits, guinea pigs, hamsters, pigs, sheep, and ferrets.

By a "genetically-modified animal cell" containing a mutant p85 gene is meant an animal cell, including a human cell, created by genetic engineering to contain a mutant p85 gene, as well as daughter cells that inherit the mutant p85 gene. These cells may be genetically-modified in culture according to any standard method known in the art. The animal cells of the invention may be obtained from primary cell or tissue preparations as well as culture-adapted and/or transformed cell lines. These cells and cell lines are derived, for example, from endothelial cells, epithelial cells, islets, neurons and other neural tissue-derived cells, mesothelial cells, osteocytes, lymphocytes, chondrocytes, hematopoietic cells, immune cells, cells of the major glands or organs (e.g., liver, lung, heart, stomach, pancreas, kidney, and skin), muscle cells (including cells from skeletal muscle, smooth muscle, and cardiac muscle), exocrine or endocrine cells, fibroblasts, and embryonic and other totipotent or pluripotent stem cells (e.g., ES cells, ES-like cells, and embryonic germline (EG) cells, and other stem cells, such as progenitor cells and tissue-derived stem cells). The preferred genetically-modified cells are ES cells, more preferably, mouse or rat ES cells, and, most preferably, human ES cells.

By "exogenous gene" is meant a gene that is added to the genome of a cell or organism. An exogenous gene may be derived from the same or different species as the genome to which it is added. An exogenous gene does not replace its counterpart gene in the genome of the cell or animal to which it is added.

By an "ES cell" or an "ES-like cell" is meant a pluripotent stem cell derived from an embryo, from a primordial germ cell, or from a teratocarcinoma, that is capable of indefinite self renewal as well as differentiation into cell types that are representative of all three embryonic germ layers.

By "insulin responsive cell" is meant a cell that expresses the insulin receptor and the Glut4 glucose transporter, such as skeletal muscle cells, cardiac cells, brown and white adipose cells, glomerular cells, renal tubular cells, and certain cells in the brain.

By "insulin responsive cell specific promoter" is meant a promoter that drives the expression of its appended gene primarily only if it is within is an insulin responsive cell. Thus, in a transgenic animal containing a DNA construct wherein the construct has a gene under the control of an insulin responsive cell specific promoter, the gene will primarily only be expressed within insulin responsive cells of the animal, and will not be significantly expressed within other cell types of the animal. Preferred promoters include the Glut4 promoter, the myosin light chain promoter, the creatine kinase promoter, the aP2 promoter, the alpha cardiac myosin heavy chain promoter, the uncoupling protein 3 promoter, the melanin-concentrating hormone promoter, the neuron-specific enolase promoter, the prion promoter, the Thy-1 promoter, the platelet-derived growth factor promoter, the synapsin promoter, and the nestin promoter and enhancer. Most preferred is the Glut4 promoter.

By "reduced" is meant a statistically significant decrease (i.e., p<0.1).

By "increased" is meant a statistically significant increase (i.e., p<0.1).

By "modulates" is meant a statistically significant increase or decrease (including a complete elimination).

Other features and advantages of the invention will be apparent from the following detailed description and from the claims. While the invention is described in connection with specific embodiments, it will be understood that other changes and modifications that may be practiced are also part of this invention and are also within the scope of the appendant claims. This application is intended to cover any equivalents, variations, uses, or adaptations of the invention that follow, in general, the principles of the invention, including departures from the present disclosure that come within known or customary practice within the art. Additional guidance with respect to making and using nucleic acids and polypeptides is found in standard textbooks of molecular biology, protein science, and immunology (see, e.g., Davis et al., *Basic Methods in Molecular Biology,* Elsevier Sciences Publishing, inc., New York, NY (1986); Hames et al., *Nucleic Acid Hybridization,* IL Press (1985); Molecular Cloning, Sambrook et al., *Current Protocols in Molecular Biology,* Eds. Ausubel et al., John Wiley and Sons; *Current Protocols in Human Genetics,* Eds. Dracopoli et al., John Wiley and Sons; *Current Protocols in Protein Science,* Eds. John E. Coligan et al., John Wiley and Sons; and *Current Protocols in Immunology,* Eds. John E. Coligan et al., John Wiley and Sons). All publications cited in this document are herein incorporated by reference.

The living system according to the invention may comprise a transgenic animal. A transgenic animal is one in whose genome a heterologous DNA sequence has been introduced. In particular, the transgenic animal is a transgenic non-human mammal, mammals being generally provided with appropriate signal transduction pathways. For the present purpose, it is generally preferred to employ smaller mammals, e.g., rabbits or rodents such as mice or rats.

For expression of the mutant regulatory p85 subunit of PI3K gene of the invention in transgenic animals, a DNA sequence encoding the mutant regulatory p85 subunit of PI3K is operably linked to additional DNA sequences required for its expression to produce expression units. Such additional sequences include a promoter as indicated herein, as well as sequences providing for termination of transcription and polyadenylation of mRNA. Construction of the expression unit for use in transgenic animals may conveniently be done by inserting a mutant DNA sequence encoding the regulatory p85 subunit of PI3K into a vector containing the additional DNA sequences, although the expression unit may be constructed by essentially any sequence of ligations.

To facilitate detection of mp85 protein, it is preferred to epitope tag the amino terminus so that it can be readily detected by immunoblotting. This is not expected to inhibit the binding of the SH2 domains to phospho-IRS-1 because it has previously been shown that a GST fusion protein that contains both p85 SH2 domains has nanomolar affinity for bifunctional synthetic phosphopeptides derived from IRS-1 containing two YMXM consensus sequences for binding to SH2 domains (Herbst et al., *Biochem.,* 33:9376-81 (1994)). Thus, the amino terminus can tolerate additional protein without affecting binding. Presently preferred epitope tags include myc, influenza hemagglutinin protein (HA), FLAG® epitope, vesicular stomatitis virus glycoprotein (VSV-G), major capsid protein of the T7 phage (T7), and poly-histidine (H₆ or H₁₀), with myc being the presently most preferred epitope tag, partially since it is much smaller than GST. Further information on myc tags and methods of using them can be found at Evans et al., *Mol. Cell. Biol.,* 5:3610 (1985) and Kolodziej et al., *Methods Enzymology,* 194:508-19 (1991). Of course, epitope tags may be located at any position within the protein, as long as they do not interfere significantly with the activity of the protein, and are not masked by their location within the protein. Thus, epitope tags may be located at the amino-terminus, carboxyl-terminus, or internally within the protein. Typically, terminally located tags are preferred.

The expression unit is then introduced into fertilized ova or early-stage embryos of the selected host species. Introduction of heterologous DNA may be carried out in a number of ways, including microinjection (cf., U.S. Pat. No. 4,873,191), retroviral infection (cf., Jaenisch, *Science,* 240:1468-74 (1988)), or site-directed integration using embryonic stem cells (reviewed by Bradley et al., *Bio*/*Technology*, 10:534-39 (1992)). The ova are then implanted into the oviducts or uteri of pseudopregnant females and allowed to develop to term. Offspring carrying the introduced DNA in their germ line can pass the DNA on to their progeny, allowing the development of transgenic populations.

General procedures for producing transgenic animals are known in the art, cf., for instance, Hogan et al., *Manipulating the Mouse Embryo: A Laboratory Manual,* Cold Spring Harbor Laboratory, 1986; Simons et al., *Bio*/*Technology,* 6:179-83 (1988); Wall et al., *Biol. Reprod.,* 32:645-51 (1985); Buhler et al., *Bio*/*Technology,* 8:140-43 (1990); Ebert et al., *Bio*/*Technology,* 6:179-83 (1988); Krimpenfort et al., *Bio*/*Technology,* 9:844-47 (1991); Wall et al., *J. Cell. Biochem.,* 49:113-20 (1992); U.S. Pat. No. 4,873,191, U.S. Pat. No. 4,873,316; WO 88/100239, WO 90/105188; WO 92/111757; and GB 87/100458. Techniques for introducing heterologous DNA sequences into mammals and their germ cells were originally developed in the mouse. See, e.g. Gordon et al., *PNAS,* 77:7380-84 (1980), Gordon and Ruddle, *Science,* 214:1244-46 (1981); Palmiter and Brinster, *Cell,* 41:343-45 (1985); Brinster et al., *PNAS,* 82:4438-42 (1985); and Hogan et al. (ibid.). In brief, in the most efficient route used to date in the generation of transgenic mice, several hundred linear molecules of the DNA of interest are injected into one of the pro-nuclei of a fertilized egg according to techniques which have become standard in the art. Injection of DNA into the cytoplasm of a zygote can also be employed. Similar procedures may be employed to produce transgenic individuals of other species.

The present invention provides genetically-modified, non-human mammals that are either heterozygous or homozygous for a genetic modification that introduces a mutant p85 gene. The present invention also provides genetically-modified animal cells, including human cells, that are heterozygous or homozygous for a modification that introduces a mutant p85 gene. The animal cells may be derived by genetically engineering cells in culture, or, in the case of non-human mammalian cells, the cells may be isolated from the above-described genetically-modified, non-human mammals.

### Genetically-Modified, Non-human Mammals and Animal Cells

The above-described methods for genetic modification can be used to introduce a mutant p85 gene into virtually any type of somatic or stem cell derived from an animal. Genetically-modified animal cells of the invention include, but are not limited to, mammalian cells, including human cells. These cells may be derived from genetically engineering any animal cell line, such as culture-adapted, tumorigenic, or transformed cell lines, or they may be isolated from a genetically-modified, non-human mammal carrying the desired mutant p85 gene.

Preferred genetically-modified animal cells are ES cells and ES-like cells. These cells are derived from the preimplantation embryos and blastocysts of various species, such as mice (Evans et al., *Nature,* 129:154-56 (1981); Martin, *PNAS,* 78:7634-38, (1981)), pigs and sheep (Notanianni et al., *J. Reprod. Fert.,* Suppl. 43:255-60, (1991); Campbell et al., *Nature,* 380:64-68 (1996)), and primates, including humans (Thomson et al., U.S. Patent No. 5,843,780; Thomson et al., *Science,* 282:1145-47 (1995); and Thomson et al., *PNAS,* 92:7844-48 (1995)).

These types of cells are pluripotent. That is, under proper conditions, they differentiate into a wide variety of cell types derived from all three embryonic germ layers: ectoderm, mesoderm and endoderm. Depending upon the culture conditions, a sample of ES cells can be cultured indefinitely as stem cells, allowed to differentiate into a wide variety of different cell types within a single sample, or directed to differentiate into a specific cell type, such as macrophage-like cells, hepatocytes, pancreatic β-cells, neuronal cells, cardiomyocytes, chondrocytes, adipocytes, smooth muscle cells, endothelial cells, skeletal muscle cells, keratinocytes, and hematopoietic cells, such as eosinophils, mast cells, erythroid progenitor cells, or megakaryocytes. Directed differentiation is accomplished by including specific growth factors or matrix components in the culture conditions, as further described, for example, in Keller et al., *Curr. Opin. Cell Biol.,* 7:862-69, (1995); Li et al., *Curr. Biol.,* 8:971 (1998); Klug et al., *J. Clin. Invest.,* 98:216-24 (1996); Lieschke et al., *Exp. Hematol.,* 23:328-34 (1995); Yamane et al., *Blood,* 90:3516-23 (1997); and Hirashima et al., *Blood,* 93:1253-63 (1999).

Genetically-modified murine ES cells may be used to generate genetically-modified mice. Embryonic stem cells are manipulated according to published procedures (Robertson, *Teratocarcinomas and Embryonic Stem Cells: A Practical Approach,* Ed. E. J. Robertson, Oxford: IRL Press, pp. 71-112 (1987); Zjilstra et al., *Nature,* 342:435-38 (1989); and Schwartzberg et al., *Science,* 246:799-803 (1989)). The particular embryonic stem cell line employed is not critical; exemplary murine ES cell lines include AB-1 (McMahon and Bradley, *Cell,* 62:1073-85 (1990)), E14 (Hooper et al., *Nature,* 326:292-95 (1987)), D3 (Doetschman et al., J. *Embryol. Exp. Morph.,* 87:27-45 (1985)), CCE (Robertson et al, *Nature,* 323:445-48 (1986)), RW4 (Genome Systems, St. Louis, MO), and DBA/1lacJ (Roach et al., *Exp. Cell Res.,* 221:520-25 (1995)).

Following confirmation that the ES cells contain the desired mutant p85 gene, these ES cells are then injected into suitable blastocyst hosts for generation of chimeric mice according to methods known in the art (Capecchi, *Trends Genet.,* 5:70 (1989)). The particular mouse blastocysts employed in the present invention are not critical. Examples of such blastocysts include those derived from C57BL/KsJ mice, C57BL6 mice, C57BL6 Albino mice, Swiss outbred mice, CFLP mice, and MFI mice. Alternatively, ES cells may be sandwiched between tetraploid embryos in aggregation wells (Nagy et al., *PNAS,* 90:8424-28 (1993)).

The blastocysts containing the genetically-modified ES cells are then implanted in pseudopregnant female mice and allowed to develop *in utero* (Hogan et al., *Manipulating the Mouse Embryo: A Laboratory Manual,* Cold Spring Harbor Laboratory, 1988; and *Teratocarcinomas and Embryonic Stem Cells: A Practical Approach,* E.J. Robertson, ed., IRL Press, Washington, D.C., 1987). The offspring born to the foster mothers may be screened to identify those that are chimeric for the mutant p85 gene. Such offspring contain some cells that are derived from the genetically-modified donor ES cell as well as other cells from the original blastocyst. Offspring may be screened initially for mosaic coat color where a coat color selection strategy has been employed to distinguish cells derived from the donor ES cell versus the other cells of the blastocyst. Alternatively, DNA from tail tissue of the offspring can be used to identify mice containing the genetically-modified cells.

The mating of chimeric mice that contain the mp85 gene in germ line cells produces progeny that possess the mp85 gene in all germ line cells and somatic cells. Mice that are heterozygous for the mp85 gene can then be crossed to produce homozygotes (see, e.g., U.S. Pat. No. 5,557,032, and U.S. Pat. No. 5,532,158).

An alternative to the above-described ES cell technology for transferring a genetic modification from a cell to a whole animal is to use nuclear transfer. This method is not limited to making mice; it can be employed to make other genetically-modified, non-human mammals, for example, sheep (McCreath et al., *Nature,* 29:1066-69 (2000); Campbell et al., *Nature,* 389: 64-66 (1996); and Schnieke et al., *Science,* 278:2130-33 (1997)) and calves (Cibelli et al., *Science,* 280:1256-58, (1998)). Briefly, somatic cells (e.g., fibroblasts) or pluripotent stem cells (e.g., ES-like cells) are selected as nuclear donors and are genetically-modified to contain a mutant p85 gene. Nuclei from donor cells which have the appropriate mp85 gene are then transferred to fertilized or parthenogenetic oocytes that are enucleated (Campbell et al., *Nature,* 380:64 (1996); Wilmut et al., *Nature,* 385:810 (1997)). Embryos are reconstructed, cultured to develop into the morula/blastocyst stage, and transferred into foster mothers for *in utero* full term development.

The present invention also encompasses the progeny of the genetically-modified, non-human mammals and genetically-modified animal cells. While the progeny are heterozygous or homozygous for the genetic modification, they may not be genetically identical to the parent non-human mammals and animal cells due to mutations or environmental influences that may occur in succeeding generations.

The transgenic animals and cell lines produced according to the present invention are useful in methods of screening for new drugs. For example, the p85DN transgenic mice (see Example 2) can be screened for drugs that reduce the body fat composition of these mice, making them more like their wild-type counterparts. For example, changes in body fat composition of animals can be measured by DEXA analysis (see Example 3), CAT scans (high resolution x-ray microcomputed tomography, or microCT, preferably the MicroCT devices and methods of ImTek, Inc., Oak Ridge, TN), correlation to plasma leptin levels, as well as by direct measurement. Likewise, certain biological and enzymatic markers can be followed in the transgenic animals and cells produced according to the invention, and the effect of test compounds on these markers measured. Markers include plasma leptin levels, beta-hydroxybutyrate, triglyceride, free fatty acids, cholesterol, lactate, glucose, insulin, and corticosterone. Examples of cell assays include, but are not limited to, assessing lipogenesis, lipolysis and leptin secretion in adipocytes. Differentiation of pre-adipocytes into adipocytes can be assessed by quantitating intracellular triglyceride accumulation or measuring the induction of adipocyte-specific genes such as resistin, aP2, and ACRP 30. Differentiation of myoblast cells into myocytes can be assessed by measuring the induction of muscle-specific genes such as myogenin.

Examples of agents that are screened include, but are not limited to, nucleic acids (e.g., DNA, RNA, and antisense RNA), carbohydrates, lipids, proteins, antibodies, peptides, peptidomimetics, small molecules, and other agents. Agents can be selected individually for testing or as part of a library. These libraries are obtained using any of the numerous approaches in combinatorial library methods known in the art, and include: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (e.g., Lam, *Anticancer Drug Des.,* 12:145 (1997); U.S. Patent No. 5,738,996; and U.S. Patent No. 5,807,683).

Examples of methods for the synthesis of molecular libraries can be found in the art, for example, in DeWitt et al., *PNAS,* 90:6909 (1993), Erb et al., *PNAS,* 91:11422 (1994), Zuckermann et al., *J. Med. Chem.,* 37:2678 (1994), Cho et al., *Science,* 261:1303 (1993), Carrell et al., *Angew. Chem. Int. Ed. Engl.,* 33:2059 (1994), Carell et al., *Angew. Chem. Int*. *Ed. Engl.,* 33:2061 (1994), and Gallop et al., *J. Med. Chem.,* 37:1233 (1994).

Individual agents or libraries of agents may be presented in solution (*e.g.*, Houghten, *Bio*/*Techniques,* 13:412-421 (1992)), or on beads (Lam, *Nature,* 354:82-84 (1991)), chips (Fodor, *Nature,* 364:555-556 (1993)), bacteria (U.S. Patent No. 5,223,409), spores (U.S. Patent Nos. 5,571,698; 5,403,484; and 5,223,409), plasmids (Cull et al., *PNAS,* 89:1865-69 (1992)) or phage (Scott and Smith, *Science,* 249:386-90 (1990); Devlin, *Science,* 249:404-06 (1990); Cwirla et al., *PNAS,* 87:6378-82 (1990); and Felici, *J. Mol. Biol.,* 222:301-10 (1991)).

### EXAMPLES

### Example 1 - Preparation of the mp85 DNA Construct

mp85 encoding cDNA was produced by two PCR fragments from mouse cDNA using primers Xba I-p85.1B (AGCTCTAGAGTGGCAGGGCACT; SEQ ID NO:4) and Bgl II-p85.2B (TTTAGATCTGATTTCCTGGGAAG; SEQ ID NO:5) for the 5' end, and Bgl II-p85.3B (AAGAGATCTCGCGAAGGCAACG; SEQ ID NO:6) and BamHl-p85.4M (CAGCGCGGATCCGCCTCTGTTGTGC; SEQ ID NO:7) for the 3' end. After appropriate digests (Xba I/Bgl II for 5' end and Bgl II/Bam HI for 3' end), these two PCR fragments were ligated first to each other and then into the Xba I/Bam HI site of pBluescript myc. pBluescript myc is a plasmid created by digesting a c-myc epitope cassette (SEQ ID NO:8) with Sma I and ligating the resulting fragment into the Sma I site of pBluescript II SK (Stratagene, La Jolla, CA). This created a nucleotide sequence encoding a mutant p85 protein having a 36 amino acid deletion of the p110 DNA binding domain encompassing amino acids 478-513 of the p85 gene (see SEQ ID NO:3), resulting in a mp85 protein having the sequence shown in SEQ ID NO:1.

The Glut 4 promoter was cloned by PCR using Xba I linked primers at 445bp-473bp at 5' end (GAGTCTAGATCACTCTGTCG; SEQ ID NO:9) and 2511bp-2530bp at 3' end (GACTCTAGAGTCCGATGG; SEQ ID NO:10) of plasmid pG4CHIII obtained from Dr. Jeffrey Pessin from the University of Iowa. The full amplified sequence corresponds to bp122 - bp2185 of the human glut4 gene 5' flanking region sequence seen in GenBank Acc. No. M61126 (see SEQ ID NO:11 for the full amplified sequence). The PCR fragment was digested with Xba I and cloned into the Xba I site of pBluescript myc containing the mp85 gene. The entire hGH gene was cut out as a Cla I/Hind III fragment from plasmid pZAP-N (see Gladue et al., *Clin. Exp. Immunol*., 110:397-402 (1997); Seeburg, *DNA,* 1(3):239-49 (1982); and GenBank Acc. No. M13438), polished to blunt ends using T4 DNA polymerase to fill in 5' or 3' overhangs, and ligated to Eco Ri linkers containing STOP codons in three reading frames (CCGGAATTCTGATAGTAA; SEQ ID NO:12). After Eco RI digestion, the resulting fragment was cloned into the Eco RI site of pBluescript myc containing the mp85 gene plus the Glut 4 promoter (supra). The entire transgene was separated from backbone plasmid for microinjection. Before microinjection, the transgene was purified by Not I/Xho I digest, gel isolated, purified on an Elutip column (Schleicher and Schuell catalog # 27360), concentrated by ethanol precipitation, and redissolved in TE (Tris 1mM, EDTA 0.1 mM). The resulting DNA construct has the nucleotide sequence shown in SEQ ID NO:2.

### Example 2 - Production of p85DN Transgenic Mice

Pronuclear transgenic mice were produced by established protocols (Hogan et al., in *Manipulating the Mouse Embryo, A Laboratory Manual* (Cold Spring Harbor, 1986); DePamphilis et al., *Biotechniques,* 6:662-88 (1998)) using embryos obtained from the inbred FVB strain of mice. The 6.6kb (Not I - Xho I fragment) mutant mouse p85 transgene construct of Example 1 was microinjected at about 2.0 µg/ml into pronuclei of embryos derived from matings of FVB males and superovulated females (Taconic, Germantown, NY). Four hundred twenty seven injected embryos were transferred to pseudopregnant CD-1 females (Charles Rivers Laboratories, Wilmington, MA). Of 68 offspring, 12 founder transgenic mice were identified by the polymerase chain reaction (PCR) of total genomic DNA. Independent lines were established by backcrossing founder mice with FVB mates.

### Example 3 - DEXA Analysis of Body Fat Composition

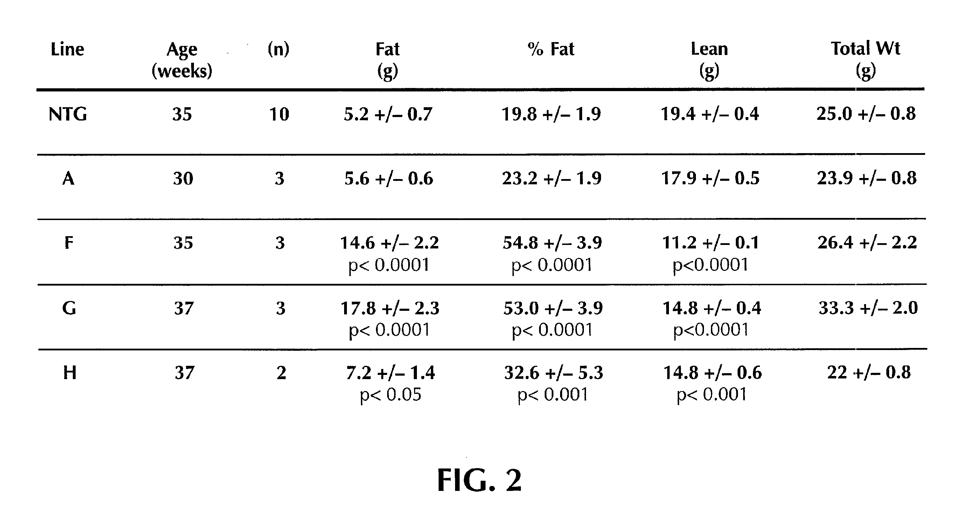

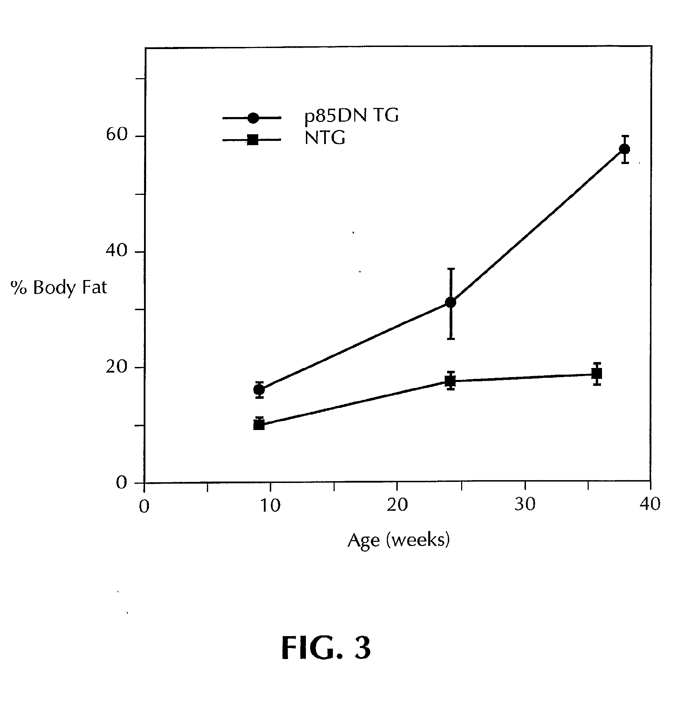

p85DN transgenic mice and their nontransgenic littermates under ketamine/xylazine anesthesia underwent dual-energy X-ray absorptiometry (DEXA, QDR-1000/W, Hologic Inc., Waltham, MA) equipped with a Whole Body Scan software for lean and fat body mass determination. Total weight, lean and fat body mass, and percent lean and fat body mass were determined (Ke et al., *Endocrinology,* 139:2068-76 (1998). In Figure 2, both wild-type mice and mice from p85DN transgenic lines A, F, G, and H are shown, and measurements were taken when the mice were between 30 and 37 weeks of age. In Figure 3, DEXA analysis was performed on mice from the non-transgenic group as well as p85DN transgenic mice from line G at various age points to determine when the body fat composition variations occurred.

Transgenic mice carrying the p85DN gene exhibited significantly higher body fat and significantly lower lean muscle mass than their wild-type counterparts. See Figures 2 and 3.

### Example 4 - Oral Glucose Tolerance Test of p85DN Transgenic Mice

Non-anesthetized p85DN transgenic mice and their nontransgenic littermates were fasted overnight and then bled via the orbital sinus (0.025 ml) immediately prior to administration of an oral glucose load (2 g glucose per kg of body weight) by gavage using a syringe equipped with a murine oral feeding needle (20 gauge; Popper & Sons, Inc., New Hyde Park, NY). The 0.025 ml blood sample was added to 0.1 ml of 0.025 % heparinized-saline in Denville Scientific microtubes (South Plainfield, NJ). The tubes were spun at the highest setting in a Beckman Microfuge 12 (Beckman Coulter, Fullerton, CA) for 2 minutes. Plasma was collected for plasma glucose determination using the VP Super System Autoanalyzer (Abbott Laboratories, North Chicago, IL). Mice were subsequently bled after 30, 75, and 120 min and plasma glucose determined in the same manner.

Transgenic mice carrying the p85DN gene exhibited the same ability to dispose of an oral glucose load as did their wild-type counterparts.

### Example 5 - Insulin Tolerance Test of p85DN Transgenic Mice

Non-anesthetized p85DN transgenic mice and their nontransgenic littermates in the fed state were bled via the orbital sinus (0.025 ml) immediately prior to administration of a subcutaneous insulin injection (2 Units/kg body weight). After injection, mice were bled at 30, 75, and 120 minutes and plasma glucose was determined as described in Example 4.

Transgenic mice carrying the p85DN gene exhibited the same response to a subcutaneous insulin injection as did their wild-type counterparts.

### Example 6 - Glycogen Content Analysis of p85DN Transgenic Mice Tissues

p85DN transgenic mice and nontransgenic mice in the fed or overnight-fasted (18h) state were killed by decapitation and then mixed hindlimb skeletal muscle, heart, and liver samples were rapidly isolated and then clamp frozen in liquid N₂ cooled aluminum tongs; tissue glycogen levels were then determined by the method of Hassid and Abraham *(Methods Enzym.,* 3:34-35 (1959)). Frozen tissue (approximately 150 mg) was cut into 20 - 30 mg blocks taking care to prevent the tissue from thawing. The frozen tissue was placed in test tubes and dissolved in 0.3 ml of 30% KOH (w/v) by immersion in boiling water for 1 hr. Glycogen was precipitated by the addition of 0.2 ml of 2% (w/v) Na₂SO₄ and 2 ml of ice-cold absolute ethanol, and the samples were stored overnight at -20°C. The next day samples were centrifuged at 3500 rpm for 10 minutes and the supernatant was discarded. The resulting pellet was washed once with 2 ml of ice-cold 66% ethanol. Glycogen was converted into monosaccharides by boiling the pellet in 1 ml of 3.79 M H₂SO₄ for 3 hrs. Following this, 0.1 ml of 0.33 M MOPS was added to each tube and the solution was neutralized with 10 M KOH to pH 7. The final volume of the solution was measured and glucose concentration was determined by using a glucose determination kit (Sigma, St. Louis, MO).

Liver, skeletal muscle, and cardiac glycogen content were the same in transgenic mice carrying the p85DN gene and their wild-type counterparts.

### Example 7 - 2-Deoxyglucose Uptake in Isolated Soleus Muscle of p85DN Transgenic Mice

Non-fasted male p85DN transgenic mice and their nontransgenic littermates weighing approximately 30 g were anesthetized via an i.p. injection of pentobarbital sodium (6.5 mg/100 g body weight). Soleus muscles (approximately 12 mg) were then isolated and incubated individually in a manner similar to that described previously (Etgen et al., *Am. J. Physiol.,* 271:E294-E301 (1996)). Briefly, soleus muscles were preincubated for 90 min at 29°C in 1.8 ml of continuously gassed (95% O₂ - 5% CO₂) Krebs-Henseleit bicarbonate buffer (KHB) containing 0.1% bovine serum albumin (BSA), 8 mM glucose, and 32 mM mannitol. After the preincubation phase, muscles were washed in KHB (1.8 ml at 29°C) containing 0.1% BSA and 40 mM mannitol. Muscles were then transferred to fresh KHB and glucose transport was measured over 20 min at 29°C in the presence of 1 mM 2-deoxy-D-glucose (2.25 mCi/mmol 2-[3H(G)]-deoxy-D-glucose), 39 mM mannitol (10 µCi/mmol [U-¹⁴C]-D-mannitol), and 2 mM sodium pyruvate. Following the final incubation phase, muscles were blotted on gauze and then clamp frozen in liquid N₂ cooled aluminum tongs. Frozen muscle was trimmed of any tendon, weighed, and digested in 0.5 ml of 1N KOH heated to 60°C for 1 hour. The digest was then cooled on ice and 0.5 ml of 1N HCI was added to neutralize it. A 0.3 ml aliquot was then added to 5 ml of Beckman Coulter Ready-Safe scintillation cocktail (Fullerton, CA) and counted on an LKB-Wallac 1219 Rack Beta scintillation counter (Perkin Elmer Wallac, Gaithersburg, MD). Glucose transport activity, expressed in µmol/g/20 min, was calculated from the intracellular 2-[3H]-deoxy-D-glucose accumulation using [U-¹⁴C]mannitol as the extracellular marker. When the effects of insulin were examined, the hormone was present during preincubation, wash, and incubation phases.

2-Deoxyglucose uptake in isolated soleus muscle under both basal or insulin-stimulated conditions was the same in transgenic mice carrying the p85DN gene as in their wild-type counterparts.

### Example 8 - Correlation of fat pad weight and plasma leptin levels

Following body composition analysis by DEXA as described above, p85DN transgenic mice and nontransgenic littermates were killed by decapitation and about 1 ml of blood was collected in Becton-Dickinson Microtainer® brand plasma separator tubes (Franklin Lakes, NJ) with lithium heparin. The tubes were spun in a Beckman Microfuge 12 at the maximum setting for five minutes. Plasma was collected in 1.5 ml Eppendorf tubes and snap frozen in liquid nitrogen. Plasma samples were stored at -80°C until analyzed for leptin levels. Plasma leptin was quantitated using an Enzyme Immuno Assay (Assay Designs Rat Leptin Correlate Kit, Ann Arbor, Ml). Gonadal fat pads were rapidly removed and then clamp frozen in liquid N₂ cooled aluminum tongs. Frozen fat pads were weighed, wrapped in aluminum foil and stored at -80°C for further analysis.

Plasma leptin levels were significantly correlated (R² = 0.949) in a positive manner with gonadal fat pad weight in transgenic mice carrying the p85DN gene and their wild-type counterparts.

## Claims

1. A genetically-modified, non-human mammal having an altered body fat composition, wherein said mammal comprises an exogenous mutant p85 P13-K gene, wherein the expression of said gene is driven by an insulin responsive cell specific promoter.

2. A mammal according to claim 1, wherein said mutant p85 P13-K gene encodes a mutant p85 P13-K protein lacking the inter-SH2 domain.

3. A mammal according to either claim 1 or 2, wherein said mutant p85 P13-K protein has the amino acid sequence of SEQ ID NO:1.

4. A mammal according to any one of claims 1 to 3, wherein said mutant p85 P13-K gene encodes a p85 P13-K protein whose inter-SH2 domain is unable to functionally interact with the p110 subunit of P13-K.

5. A mammal according to any one of claims 1 to 4, wherein said insulin responsive cell specific promoter is selected from the group consisting of the GLUT4 promoter, the myosin light chain promoter, the creatine kinase promoter, the aP2 promoter, the alpha cardiac myosin heavy chain promoter, the uncoupling protein 3 promoter, the melanin-concentrating hormone promoter, the neuron-specific enolase promoter, the prion promoter, the Thy-1 promoter, the platelet-derived growth factor promoter, the synapsin promoter, and the nestin promoter and enhancer.

6. A mammal according to any one of claims 1 to 5, wherein said mammal is a rodent.

7. A genetically-modified animal cell, wherein said cell comprises an exogenous mutant p85 P13-K gene, wherein the expression of said gene is driven by an insulin responsive cell specific promoter.

8. An animal cell according to claim 7, wherein said mutant p85 P13-K gene encodes a mutant p85 P13-K protein lacking the inter-SH2 domain.

9. An animal cell according to either claims 7 or 8, wherein said mutant p85 P13-K protein has the amino acid sequence of SEQ ID NO:1.

10. An animal cell according to any one of claims 7 to 9, wherein said insulin responsive cell specific promoter is selected from the group consisting of the GLUT4 promoter, the myosin light chain promoter, the creatine kinase promoter, the aP2 promoter, the alpha cardiac myosin heavy chain promoter, the uncoupling protein 3 promoter, the melanin-concentrating hormone promoter, the neuron-specific enolase promoter, the prion promoter, the Thy-1 promoter, the platelet-derived growth factor promoter, the synapsin promoter, and the nestin promoter and enhancer.

11. A method of identifying an agent that modulates the body fat composition of a mammal, said method comprising the steps of
comparing the body fat compositions of a test animal to a control animal, wherein said agent has been administered to said test animal, further wherein each of said test and control animals is a genetically-modified, non-human mammal having an increased body fat composition, wherein said mammal comprises an exogenous mutant p85 P13-K gene, wherein the expression of said gene is driven by an insulin responsive cell specific promoter; and
identifying as a modulating agent the agent that has a statistically significant effect on the body fat composition of the test animal.

12. A DNA expression construct for expressing a mutant p85 P13-K gene, said construct comprising:
a mutant p85 P13-K gene, wherein said gene encodes a p85 P13-K protein whose inter-SH2 domain is unable to functionally interact with the p110 subunit of P13-K; and
an insulin responsive cell specific promoter that controls the expression of said gene.

13. A DNA expression construct according to claim 12, wherein the gene encodes a mutant p85 P13-K protein lacking the inter-SH2 domain.

14. A DNA expression construct according to either claim 12 or 13, wherein said mutant p85 P13-K protein has the amino acid sequence of SEQ ID NO:1.

15. A DNA expression construct according to any one of claims 12 to 14, wherein said insulin responsive cell specific promoter is selected from the group consisting of the GLUT4 promoter, the myosin light chain promoter, the creatine kinase promoter, the aP2 promoter, the alpha cardiac myosin heavy chain promoter, the uncoupling protein 3 promoter, the melanin-concentrating hormone promoter, the neuron-specific enolase promoter, the prion promoter, the Thy-1 promoter, the platelet-derived growth factor promoter, the synapsin promoter, and the nestin promoter and enhancer.
